Europäisches Patentamt

(19) European Patent Office

Office européen des brevets

(11) Publication number: **0 084 251**

**A1**

(12) # EUROPEAN PATENT APPLICATION

(21) Application number: **82306884.6**

(22) Date of filing: **23.12.82**

(51) Int. Cl.³: **A 61 B 10/00**
A 61 B 17/22, A 61 B 1/00
A 61 B 17/32, G 01 N 1/04

(30) Priority: **28.12.81 JP 195721/81 U**

(43) date of publication of application:
**27.07.83 Bulletin 83/30**

(84) Designated Contracting States:
**AT BE CH DE FR GB IT LI NL SE**

(71) Applicant: **OLYMPUS OPTICAL CO., LTD.**
**43-2, 2-chome, Hatagaya Shibuya-ku**
**Tokyo 151(JP)**

(72) Inventor: **Mizumoto, Morihide**
**Olympus ryo, No. 22-13 Oowadamachi 4-chome**
**Hachioji-shi Tokyo(JP)**

(74) Representative: **Kennedy, Victor Kenneth et al,**
**G.F. REDFERN & CO. Marlborough Lodge 14 Farncombe**
**Road**
**Worthing West Sussex BN11 2BT(GB)**

(54) **Sampling devices for use in an endoscope.**

(57) A sampling device for use in an endoscope making it possible to easily and positively collect a predetermined amount of samples such as unwanted deposits from industrial pipes, or cell tissue from cavities within living bodies.

When liquids containing chemical substances or rusts are transmitted through pipes, unwanted deposits form on the inside walls of the pipes, and in industrial plants and machines the amounts of liquid that can be passed will be reduced and so restrict the production efficiency, and possibly lead to other troubles. Therefore, samples of such deposits must be taken from such pipes for inspection. Industrial endoscopes are often used to collect such samples.

Medical endoscopes are used to collect samples such as cell tissue from internal walls of cavities in living bodies and to inspect the body cavities.

A sample scraping means (24) is formed at the tip of a suction tube (20) retractable in a forceps inserting channel (18) formed in an endoscope (11) and connectable at the rear end to a suction device (25) so that even powdery samples may be easily and positively collected.

./...

Croydon Printing Company Ltd.

FIG.2

-1-

## SAMPLING DEVICES FOR USE IN AN ENDOSCOPE

This invention relates to sampling devices for use in endoscopes by which samples can be easily and positively collected from deposits on the inside walls of pipes or the like, or cell tissues collected from body cavities.

When liquids such as water, oil and solutions are transmitted through pipes chemical substances or rusts contained in such liquids are deposited on the inside walls of the pipes, especially in chemical plants, water purification plants and all kinds of machines. The amount of liquid that can be passed will then be reduced, and not only lead to a drop in production efficiency but also cause other troubles. Therefore, it is necessary to collect and inspect samples from the deposits on inside walls of the pipes, and to remove the deposits if cleansing proves necessary. Industrial endoscopes are often used for such collection and inspection.

Cell tissues need to be taken from the inside wall of a body cavity and collected to diagnose the state within the body cavity, using a medical endoscope for inspecting the living body cavity.

One example of the prior art used for such purposes is disclosed in the Gazette of Japanese Utility Model Publication No. 7506/1972. In this prior art example, for collecting samples cell tissue, a collecting forceps is thrust at the inside wall of a body cavity, and air is drawn in by a suction syringe through an air path provided inside the collecting forceps, to collect cells as samples.

In the sampling device of such structure, as the cells are collected by the suction action merely by the suction syringe, they will not always be collected, as in some cases the action will not be positive enough and if the object to be collected is a powder, unless the scraping and suction operations are simultaneously made, it may not be able to be collected.

Further, there has been a problem in cases where the amount required to be collected is large, or the number of collecting places are many, as sampling becomes very toilsome.

This problem is substantially the same for industrial endoscopes.

A further prior art example, wherein a tube can be connected at the rear end to a suction valve, and a single loop-shaped scraping or scratching blade is projected forward of an aperture at the tip of the tube to be a sample suction port is disclosed in the Gazette of U.S. Patent No. 4043322.

In this prior art example, as the suction port is provided on the rear base side of the loop if the scraped sample is a powder, it may fly around and cannot then be collected efficiently. Also, in the case of collecting cells of a structure in an affected part, the loop-shaped scraping blade will scrape off the cells in the form of a block and will be likely to hurt the affected part more than is necessary. Further, if the scraped sample is too large, it will not be able to be efficiently taken into the suction port. Therefore, in this prior art example, great skill is required for the sampling operation.

A further prior art example wherein a sample suction tube is inserted through a forceps inserting channel of an endoscope and is provided with a sample scraping blade and a sample suction aperture in the tip forming part arranged at the tip so that the sample may be simultaneously scraped off, drawn in and collected as is suggested by the present inventor in Japanese Patent Application No. 129666/1981.

This device has an advantage that, as the scraping blade is provided near the suction aperture, the scraped sample will not fly around and can be effectively collected.

However, as the scraping blade of this device is formed on the side surface of the tip forming part positioned at the tip of the above mentioned sample suction tube, when scraping off the sample this scraping blade will have to be contacted so that the axis of the above mentioned tip part may be parallel with the object wall surface. Therefore, in cases where the sample scraping work is to be carried out near the hand of the worker, it can be carried out efficiently but, in cases where this work is to be carried out in a remote position separated by some distance from the hand of the operator, the force thrusting the above mentioned scraping blade against the wall surface will become weaker, and therefore the capability for scraping off samples will be reduced and, in cases where the sample tightly adheres to the wall surface, it may not be readily scraped off.

Also, when the force thrusting the scraping blade on the wall surface is thus reduced, the force closely contacting the sample suction aperture provided near this scraping blade with the above mentioned wall surface will simultaneously weaken, therefore, a clearance will be made between the aperture and wall surface and the scraped sample may fly through this clearance and will not be efficiently drawn in and collected. Therefore, a large suction force will be required.

Objects of the present invention are to provide a sampling device for use in an endoscope by which samples can be positively collected, even if samples are tightly adhering to a wall surface or the like, or if samples are to be collected from a remote position separated from the operators hand.

According to one exemplary embodiment of the present invention there is provided a sampling device for use in an endoscope, wherein a sample scraping member formed at the tip of a suction tube provided with an extraction path through which pass samples together with extracted air when retractably inserted through a forceps inserting channel of an endoscope so that samples may be collected, the tip of said suction tube having an outer periphery that forms a suction port communicating with the extraction path together with said sample scraping member, which is provided by an inclined surface forming a sample scraping blade as part of the peripheral end of the suction tube, which tube extends to an external connector for attachment to a suction device.

The invention will now be described with reference to the drawings, in which:-

Figure 1 is a schematic side view showing details of a known prior art device;

Figure 2 is a longitudinal sectional view showing details of a first exemplary embodiment together with an endoscope;

Figure 3 is a longitudinal sectional view showing details of the embodiment shown in Figure 2 in magnified form;

Figure 4 is a longitudinal sectional view showing a second exemplary embodiment of the present invention; and

Figure 5 is a longitudinal sectional view showing a third exemplary embodiment of the present invention.

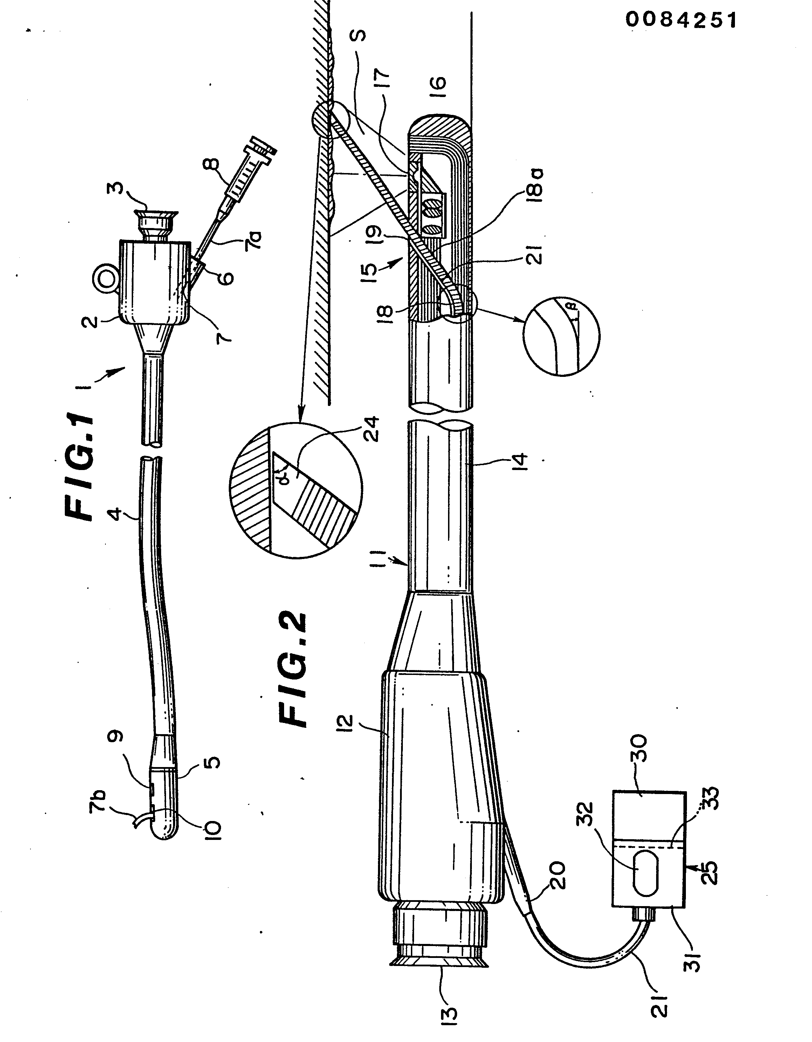

Prior to explaining the respective embodiments of the present invention, the conventional example of a sampling device used in a known endoscope disclosed in the Gazette of Japanese Utility Model Publication No. 7506/1972 will be described with reference to Figure 1.

A medical or veterinary endoscope 1 shown in Figure 1 is intended for diagnostic examination of living body cavities, and is formed of an operating part 2, an eyepiece 3 formed at the rear end of this operating part 2, a flexible sheath 4 containing a light transmitting means and leading to a tip section 5. This endoscope 1 is provided with an internal forceps channel controlling a flexible collecting forceps 7 which is retractable and can be withdrawn from a forceps inserting port 6 of the operating part 2, or inserted to enter the tip section 5. The collecting forceps 7 has such a suction device as a syringe 8 removably fitted at its outer tip 7a on the operating side and projects at the inner end from a slot 10 near an observing window 9, so that its tip 7b can reach the inside wall of a body cavity.

If an aperture is to be a sample suction port at the tip 7b of the above mentioned collecting forceps 7 and the endoscope is perfectly closed, no air can be drawn into the collecting forceps 7 and samples such as cells or cell tissue will not be collectable. Therefore, an inlet air path is provided at the edge of the sample suction port.

When collecting object samples such as cells or cell-tissue using this collecting device, if the tip 7b of the collecting forceps 7 is thrust at the inside wall of a cavity in an object place while being observed through the observing window 9 of the endoscope 1 and air is drawn in by the suction syringe 8 through the air path provided inside the collecting forceps 7, the cells on the above mentioned inside wall will be sucked into the collecting forceps 7. When the cells have been collected, the forceps 7 is pulled out of the endoscope 1 and air is fed into the forceps 7 from its outer tip 7a so that the cells collected in the suction port may be recovered.

In a collecting device having such a construction, as the cells are only collected by the limited suction action by the syringe 8, they will not always be collectable, as in some cases positive suction will be lacking and, in cases where the object sample is a powder, unless the scraping operation and suction operation are made simultaneously, the samples will not be collectable in some cases.

Further, there has been a problem that, in case the amount required to be collected is large or in case the collecting places are many, the collecting operation will be very tedious.

This problem will be substantially the same in industrial applications, when using such a device inserted through a channel provided in an industrial endoscope.

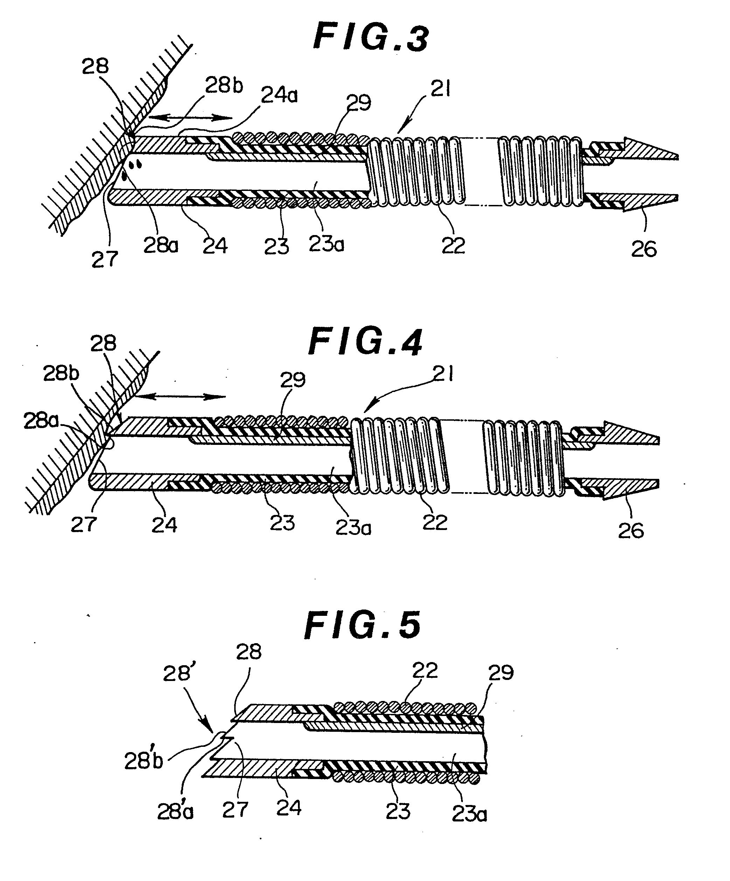

A first exemplary embodiment of the present invention will now be described with reference to Figures 2 and 3.

In these drawings, an industrial endoscope 11 is formed with an operating part 12, an eyepiece 13, a flexible insertion sheath 14 and a tip section 15.

The extreme end 16 of the tip section projects a lateral illuminating light beam from the operating part 12 via a fibre-optic bunch, and an adjacent observation window 17 is provided in the side of the tip section 15. An observing optical system is arranged within the sheath to convey the view from this window 17 to the eyepiece 13, so as to form a side sight type endoscope. A forceps outlet port 19 of a forceps inserting channel 18 forms an opening to the rear of the observing window 17 on the side surface of the tip section 15. This forceps inserting channel 18 is provided internally and extends along the insertion sheath to a forceps inserting port 20 formed, for example, on the lower surface of the endoscope operating part 12 as shown in Figure 2, and is formed with an inclined bend so as to rise diagonally forward toward the forceps projecting port 19 within the tip section. The angle of inclination of the channel 18 in the tip section is set to be within the later described range for putting the tip of an inserted forceps into the visual field S of the endoscope 11, and it is preferable that this angle of inclination of the channel 18 in the tip section is set to be within a range of from 15 to 60 degrees. In the thus formed forceps inserting channel 18, in this embodiment of the present invention, a sampling device suction tube 21 is retractably inserted via the forceps inserting port 20. As shown in Figure 3, this sampling device suction tube 21 is formed of a flexible tube 23 fitted within a spiral tube 22, an annular sample scraping member 24 being arranged at the inner tip of the tube 23 and an annular connector 26 provided at the rear end of the tube 23 so as to connect the tube 23 to a later described suction device 25. A sample suction port 27 is formed in the tip of the sample scraping member 24. The end surface including the peripheral end of the outer periphery of the sample sucking port, that is, the tip surface of the sample scraping member 24, is formed to be a surface inclined at an appropriate angle with respect to the axial direction of the tube 23. This sample suction port 27 communicates with the air path 23a of the above mentioned suction tube 23.

As shown in Figure 2, an angle $\alpha$ is formed by the inclination at the front end of this inclined tip surface, and is substantially identical to the angle $\beta$ of the inclined part 18a of the forceps inserting channel 18 so that, as described later, when the insertion sheath 14 of the endoscope is inserted into a pipe line of a plant or the like and the sampling device 21 is projected out of the forceps projecting port 19, the inclined tip surface will be able to closely contact the wall surface of the pipe line. A sample scraping blade 28 is provided to project over a suitable width in the peripheral direction along the peripheral edge of the rear-most part of the sample suction port 27.

This scraping blade 28 has an internal surface 28a, on the sample suction port 27 side, forming a tapered projecting surface at the peripheral end of the surface, and has an outer surface 28b forming a surface projecting sharply so as to be flush with the outer peripheral surface 24a of the scraping member 24, so that when this scraping blade 28 is thrust in an inclined direction against the inside wall of an object, samples on the surface of the inside wall will be scraped off by the tapered peripheral tip of the scraping blade 28.

Furthermore, on the inner periphery of the above mentioned suction tube 23, a wire 29 is provided between the tip part and the connector 26 so that the strength of the tube 23 may be increased and the scraping blade 28 may be strongly thrust against a desired part on the wall surface.

The above mentioned connector 26 is substantially tubular, is formed with a taper towards the rear end from the base part on the outer periphery and is made resilient in the rear end portion, so as to return outwardly after being pressed together during connection to a suction device 25.

As shown in Figure 2, this suction device is formed of a suction pump 30 and a specimen chamber 31. The suction pump 30 contains suction means for air or liquid and samples contained therein. The chamber 31 is provided with an indicator 32 by which the amounts (and collected samples) drawn out and collected in this chamber can be observed, and a filter 33 stops collected samples from entering the suction pump 30.

In collecting samples using the first described exemplary embodiment, first of all the inserted sheath 14 of the endoscope is inserted through the inlet of a pipe line, and a light source provides illumination so that a view can be seen through the observation window 17 of the inserted sheath 14, so that the inside wall of the pipe line is observed. If a deposit is discovered on the inside wall, the sampling device suction tube 21 will be inserted into the forceps inserting channel 18 through the forceps inserting port 20. Then, as this forceps inserting channel 18 is formed to bend within the tip section 15 of the endoscope 11 so that it is directed toward the visual field of the endoscope 11 through the inclined part 18a, the sampling device suction tube 21 will be projected from the tip section so that the scraping member 24 passes out through the port 19, being regulated by the inclined part 18a. When the external suction tube 23 section that extends from the forceps inserting port 20 to the connector 26 is manually operated to further advance the sampling device tube 21, the scraping member 24 will enter the visual field S of the endoscope 11 and finally reach the wall surface of the pipe line. Then, when the external portion of the tube 23 is manually operated to move forward and rearward, the scraping member 24 will be moved forward and rearward in the axial direction of the tube 23 to scrape off the deposit adhering to the wall surface of the pipe line. In such case, when the suction device 25 is operated, as this scraped sample of the deposit will be drawn together with air into the suction port 27, and be passed through the extraction air path 23a of the suction tube 23 and drawn into the subsequent suction device 25. At this time, the scraping blade 28 will be thrust in an inclined direction against the wall surface, therefore a component thrusting the scraping blade 28 vertically onto the wall surface will be produced, and the deposit can be efficiently scraped off with a strong sample scraping force. Also, the sample suction port 27 can be strongly thrust into close contact with the wall surface by this component, so that there is little clearance from the wall surface, and the scraped sample will not fly around but will be efficiently drawn away and removed.

Figure 4 is a longitudinal sectional view showing a second exemplary embodiment in which a modified form of scraping member 24 is fitted to the sampling device suction tube 21. In this embodiment, the rear inner surface 28a of the sample suction port 27 of the scraping blade 28 is formed by a surface forward projecting sharply at the inner peripheral surface, and the outer surface 28b is formed by a slowly tapering surface.

Figure 5 is a longitudinal sectional view showing a third exemplary embodiment in which a differently modified scraping member 24 is fitted to the sampling device suction tube 21. In this embodiment there is provided a plurality of additional scraping blades 28', each similar in form to the scraping blade 28 of the second embodiment, in addition to the rearmost scraping blade 28. These additional scraping blades 28' surround the peripheral edge of the sample suction port 27 and all project sharply downward toward the front. In this embodiment, as many scraping blades 28' are provided on the peripheral edge of the sample suction port 27, to be simultaneously thrust at many places on the object wall surface, higher efficiency may be achieved in the scraping off of samples.

The scraping blades that may be used in embodiments of the present invention are not limited to the above described scraping blades, 28 and 28', but may be of various shapes. Further, the device can be used not only to collect samples as described above but also to cleanse the wall surfaces of objects such as pipes by scraping off unwanted deposits and to remove them by suction. In such cases, the device may be made larger, to function more efficiently.

In each of the above described embodiments, as the scraping blade projects no more than is necessary from the peripheral edge of the scraping member 24, the wall surface is not likely to be extensively damaged by the scraping operation. Therefore, the present invention will be effective not only for collecting samples from such wall surfaces as pipe interiors, but can also be adapted for use in the medical or veterinary fields, for collecting samples such as cell tissue structures from affected parts in living body cavities.

In the case of collecting samples or removing deposits from a pipe wall surface or the like by using the device of the present invention, if the samples or deposits are scraped off with the sharply projecting part of the scraping blade 28 formed on the peripheral edge at the rear of the suction port 27 while keeping the front tip of the scraping member 24 adjacent to but spaced from the wall surface, samples can be taken not only by advancing and retreating the tube 21 forward and rearward by hand, but also by moving the endoscope 11 forward with the tube held in place, as shown, for example, in Figure 2, as samples can be continuously and efficiently scraped off, extracted by suction and collected.

In the above mentioned embodiments, the present invention has been applied to a side sight type endoscope, but it can be applied to straight sight type and inclined sight type endoscopes.

Furthermore, in some cases embodiments of the present invention can be used along, without any endoscope, where it is necessary to clean or examine a position on a wall surface near an opening, for example.

As described above, embodiments of the present invention provide advantages, as the sample collecting scraping blade is thrust against the wall surface to which deposits adhere in an inclined manner and therefore a force strongly thrusting the scraping blade on the wall surface will be generated, stronger than in a case where the scraping blade is thrust so that the axis of the scraping member to which the scraping blade is attached is parallel with the wall surface, and as the sample suction port is immediately adjacent to the scraping blade, the scraped samples can be effectively extracted and collected.

As the sample suction port is formed with an inclined surface, and therefore it is easy to closely contact the wall surface and, due to the component thrusting the sample suction port against the wall surface, the sample suction port will be in close contact with the wall surface, and therefore the scraped samples can be prevented from flying free and the sample recovery rate is high.

As the sampling device suction tube 21 must be a free fit within the passageway 18 and port 19 of an endoscope with which it is to be used, air is free to enter to replace the extracted air and maintain an adequate flow for positive collection of samples.

CLAIMS:-

1.  A sampling device for use in an endoscope, wherein a sample scraping member formed at the tip of a suction tube provided with an extraction path through which pass samples together with extracted air when retractably inserted through a forceps inserting channel of an endoscope so that samples may be collected, the tip of said suction tube having an outer periphery that forms a suction port communicating with the extraction path together with said sample scraping member, which is provided by an inclined surface forming a sample scraping blade as part of the peripheral end of the suction tube, which tube extends to an external connector for attachment to a suction device.

2.  A sampling device for use in an endoscope, as claimed in Claim 1, in which said sample scraping blade is formed on an obtuse angled side of the outer periphery of the suction port.

3.  A sampling device for use in an endoscope, as claimed in Claim 1, in which said sample scraping blade is formed to project from the peripheral end of the suction port.

4.  A sampling device for use in an endoscope, as claimed in Claim 1, said tip surface sample scraping blade is formed by an inclined surface having an angle of from 15 to 60 degrees.

0084251

**FIG.1**

**FIG.2**

**FIG.3**

**FIG.4**

**FIG.5**

# European Patent Office

## EUROPEAN SEARCH REPORT

| | DOCUMENTS CONSIDERED TO BE RELEVANT | | EP 82306884.6 |
|---|---|---|---|

| Category | Citation of document with indication, where appropriate, of relevant passages | Relevant to claim | CLASSIFICATION OF THE APPLICATION (Int. Cl. ³) |
|---|---|---|---|
| X | US - A - 3 173 414 (A. GUILLANT) | 1,3,4 | A 61 B 10/00 |
| A | * Fig. 2-4; column 2, line 39 - column 3, line 55 * | 2 | A 61 B 17/22 |
| | | | A 61 B  1/00 |
| | -- | | A 61 B 17/32 |
| A | US - A - 2 715 899 (K.S. MC LEAN) | 1,3,4 | G 01 N  1/04 |
| | * Totality * | | |
| | -- | | |
| A | DE - C - 561 101 (I.C. MIERLEY) | 1,4 | |
| | * Totality * | | |
| | -- | | |
| A,D | US - A - 4 043 322 (R.R. ROBINSON) | 1,3,4 | |
| | * Totality * | | |
| | -- | | |
| A | US - A - 4 016 882 (A. BROADWIN et al.) | 1,4 | TECHNICAL FIELDS SEARCHED (Int. Cl. ³) |
| | * Fig. 1,2,4; column 4, lines 41-65 * | | A 61 B  1/00 |
| | -- | | A 61 B 10/00 |
| A | US - A - 4 224 929 (H. FURIHATA) | | A 61 B 17/00 |
| | * Fig. 3,5; column 3, lines 10-44 * | | G 01 N  1/00 |
| | -- | | G 02 B 23/00 |
| D,E | EP - A2 - 0 072 689 (OLYMPUS OPT.) | | |
| | * Totality * | | |
| | ---- | | |

The present search report has been drawn up for all claims

| Place of search VIENNA | Date of completion of the search 18-04-1983 | Examiner LUDWIG |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82